# EUROPÄISCHE PATENTANMELDUNG

(11) **EP 1 955 651 A1**
(43) Veröffentlichungstag der Anmeldung: **13.08.2008**
(21) Anmeldenummer: 07002473.2
(22) Anmeldetag: 06.02.2007
(51) Int. Cl.: A61B 5/11, A61N 5/10

(54) **Vorrichtung zur räumlichen Lokalisation eines bewegbaren Körperteils**

(71) Anmelder: Siemens Schweiz AG, 8047 Zürich (CH)
(72) Erfinder: Broennimann, Thomas, 4704 Wolfisberg (CH); Cloutot, Laurent, 8956 Killwangen (CH)
(74) Vertreter: Fischer, Michael

(57) **Zusammenfassung**

Es wird eine Vorrichtung zur räumlichen (ein-, zwei oder dreidimensionalen) Lokalisation eines bewegbaren Körperteils vorgeschlagen, bei welcher der Körperteil (z.B. ein Tumor) sich innerhalb eines Bewegungsvolumens an der Oberfläche bis auch in Innerem eines lebenden Wesens (z.B. eines Menschen) befindet.

Die erfindungsgemässe Vorrichtung weist folgende Komponenten auf:
- mindestens eine optische Aufnahmevorrichtung, welche außerhalb des Wesens angeordnet ist,
- mindestens ein dosierbarer Fluorophor, welcher im Bereich des Körperteils einbringbar ist,
- eine extrinsische Strahlungsquelle, welche außerhalb des Wesens angeordnet ist und aus welcher sich eine Strahlung in Richtung des Bewegungsvolumens ausbreitet, wodurch eine spektralen Erregung des Fluorophors stattfindet, indem eine vom Fluorophor emittierte Welle erzeugt ist und bei mindestens einer von der optischen Aufnahmevorrichtung messbaren Wellenlänge ermittelbar ist,
- die optische Aufnahmevorrichtung mindestens eine optische Achse aufweist, welche in Richtung des Körperteils und dessen Bewegungsvolumens orientierbar ist,
- die optische Aufnahmevorrichtung mindestens einen zu der optischen Achse senkrechten optisch-elektrischen Wandler aufweist, welcher ein Ausgangsignal abgibt, aus welchem ein Abstand zwischen dem Fluorophor und einem Referenzpunkt ermittelbar ist.

## Beschreibung

Die Erfindung betrifft eine Vorrichtung zur räumlichen Lokalisation eines bewegbaren Körperteils nach dem Oberbegriff des Anspruches 1.

Bei einer Bestrahlungstherapie eines Tumors mittels z.B. einer Gamma- oder eine Protonenbestrahlung oder bei einer Abbildung eines Tumors im Inneren des Körpers eines Wesens existieren zahlreiche Methoden oder Vorrichtungen, bei welchen es angestrebt wird, eine möglichst genaue und reproduzierbare dreidimensionale Lokalisation des Tumors oder allgemeiner eines bewegbaren Körperteils zu gewährleisten. Einige Methoden ("in-vivo"-Abbildungsmethoden z.B. mittels einer Radiographie mit X-Strahlung) sind aber als schädliche ("invasive") Methoden eingestuft und sollten dadurch nur in eingeschränkter Weise verwendet werden. Auch weniger schädliche "in-vivo"-Methoden, wie z.B. eine Abbildungsmethode mittels magnetischer Resonanz, bleiben zusätzlich zu ihrer Komplexität, ihrem Aufwand und ihren Kosten mit und gleichzeitig zu einer Bestrahlungsbehandlung schwer kombinierbar. Andere Methoden ("ex-vivo") nutzen (z.B. optisch) detektierbare Merlamale/Markierungen, welche an der "Oberfläche" eines Patienten relativ zu einem bekannten Koordinatensystem anbringbar/eintragbar und erkennbar sind. Jedoch leiden diese Methoden an Genauigkeit und sowieso an Reproduzierbarkeit, weil physiologische Änderungen des Patienten (z.B. Gewichtabnahme) oder unvermeidliche (am schlimmsten unerwartete) Bewegungen (z.B. durch den Atem, durch ein Zittern, etc.) während Behandlungen bzw. Neupositionierungen des Patienten/Körperteilen zwischen Behandlungen auftreten. Es existieren ferner Verfahren/Vorrichtungen zur Festhaltung des Patienten oder eines Körperteils an z.B. einem Liegetisch bei der Bestrahlungstherapie oder Messeinrichtungen des Atems, jedoch verringern diese Lösungen einerseits den "Komfort" des Patienten bzw. ermöglichen keine 100%-genaue räumliche Festlegung/Lokalisation eines Tumors in einem bekannten Koordinatensystem.

Neulich konnte jedoch ein Tumor innerhalb eines kleinen Tiers (Maus) mittels einer der Maus extern angeordneten CCD-Kamera abgebildet werden. Dabei wurde in eine Blutbahn der Maus ein Fluorophor (Hematoporphyrin)injiziert, welcher aufgrund einer Tumoraffinität sich selektiv in Tumoren akkumulieren kann. Dazu wird Licht aus einer extrinsischen (d.h. außerhalb der Maus angeordneten) Lichtquelle im rot/infrarot-spektralen Wellenlängenbereich die Maus oder zumindest einen den Tumor umfassenden Teil der Maus beleuchtet. Dadurch, dass das emittierte Licht. Weichteile der Maus bis zur Stelle des Fluorophors eindringen kann und dass der Fluorophor dementsprechend dosiert wurde, konnte ein aus dem erregten Fluorophor "Auto-Fluoreszenz-Emission"-Lichtsignal als Rückstrahlung- bzw. -streuung von der CCD-Kamera rückgewonnen und gemessen werden. Damit ist es möglich geworden, ein "in-vivo"-Intensitätsbild des Tumors mit sehr geringen bzw. keinen Schadengefahren zu realisieren. Je nach den Merkmalen des Bildes des Tumors oder durch Verwendung von geeigneten Filtern können daher Aussagen über die Form oder den Typ der ungesunden Zellen gemacht werden. Eine derartige Methode ist ausfürlicher beschrieben in der Literaturstelle: L. Celentano, P. Laccetti, R. Liuzzi, G. Mettivier, M. C. Montesi, M. Autiero, P. Riccio, G. Roberti, P. Russo, Member, IEEE*,* and M. Salvatore, "Preliminary Tests of a Prototype System for Optical and Radionuclide Imaging in Small Animals", IEEE TRANSACTIONS ON NUCLEAR SCIENCE, VOL. 50, Nr. 5, OCTOBER 2003, Seiten 1693-1701. Diese attraktive, da nicht hoch schädliche ,,in vivo"-Sichtmethode eignet sich sehr gut für kleine Tiere, jedoch dabei werden auch keine Hinweise über eine genaue, räumliche (z.B. metrische) Lokalisation eines bewegenden Tumors gegeben, da das aktuelle Verfahren ein reines zweidimensionales "Planar-Imaging" von auto-fluoreszierenden Tumoren erlaubt.

Der Erfindung liegt eine Aufgabe zugrunde, eine Vorrichtung zur genauen räumlichen Lokalisation eines bewegbaren Körperteils (Tumor, Karzinom, etc) eines lebenden Wesens (d.h. auch eines menschlichen Patienten) anzugeben.

Ferner sollte die Vorrichtung derart konzipiert werden, dass eine potentielle Gefahr mit belastenden (= invasiven) Strahlungen des Patienten vermieden wird. Ebenfalls sollte die Vorrichtung gleichzeitig während einer Bestrahlungstherapie eines Patienten z.B. zur Zerstörung eines Tumors oder eines Karzinoms möglichst in Echtzeit und dreidimensional "in-vivo" lokalisieren können.

Erfindungsgemäß wird die Aufgabe durch die Merkmale des Anspruches 1 gelöst.

Es wird eine Vorrichtung zur räumlichen (ein-, zwei oder dreidimensionalen) Lokalisation eines bewegbaren Körperteils vorgeschlagen, bei welcher der Körperteil (z.B. ein Tumor) sich innerhalb eines Bewegungsvolumens an der Oberfläche bis auch in Innerem eines lebenden Wesens (z.B. eines Menschen) befindet. Die erfindungsgemässe Vorrichtung weist folgende Komponenten auf:
- mindestens eine optische Aufnahmevorrichtung, welche außerhalb des Wesens angeordnet ist,
- mindestens ein dosierbarer Fluorophor, welcher im Bereich des Körperteils einbringbar ist,
- eine extrinsische Strahlungsquelle, welche außerhalb des Wesens angeordnet ist und aus welcher sich eine Strahlung in Richtung des Bewegungsvolumens ausbreitet, wodurch eine spektralen Erregung des Fluorophors stattfindet, indem eine vom Fluorophor emittierte Welle erzeugt ist und bei mindestens einer von der optischen Aufnahmevorrichtung messbaren Wellenlänge ermittelbar ist,
- die optische Aufnahmevorrichtung mindestens eine optische Achse aufweist, welche in Richtung des Körperteils und dessen Bewegungsvolumens orientierbar ist,
- die optische Aufnahmevorrichtung mindestens einen zu der optischen Achse senkrechten optisch-elektrischen Wandler aufweist, welcher ein Ausgangsignal abgibt, aus welchem ein Abstand zwischen dem Fluorophor und einem Referenzpunkt ermittelbar ist. Der Referenzpunkt kann auf Wunsch in einem absoluten räumlichen Koordinatensystem gewählt werden. Als bevorzugter Referenzpunkt kann ein bekanntes "Isozentrum" einer nebengeordneten, therapeutischen Bestrahlungsanlage genommen werden. Der Referenzpunkt kann auch z.B. einen einzelnen Schnittpunkt auf der optischen Achse innerhalb von dem Bewegungsvolum bilden.

Die erfindungsgemässe Vorrichtung eignet sich sehr gut für unterschiedliche, ein-, zwei-, dreidimensionale Lokalisationsgraden, je nach dem u.a. wie viele optisch-elektrische Wandler eingesetzt werden. Dieser Aspekt wir ausführlicher im Folgenden beschrieben.

Im Allgemeinen lässt sich die Erfindung mit mehreren, interessanten Ausführungen realisieren, bei welchen:
- der optisch-elektrische Wandler der optischen Aufnahmevorrichtung eine Fotodiode oder eine Gruppe von nebengeordneten Fotodioden oder Pixeln, wie vorzugsweise in einer Kamera, ist,
- die optische Achse der Fotodiode oder der Kamera bewegbar ist, vorzugsweise mittels einer Schwenkung oder einer Drehung, derart dass die dadurch gebildeten, optischen Achsen einen positiven Winkel miteinander bilden und einen einzelnen Schnittpunkt im Bewegungsvolumen bilden.

Oder:
- die optische Aufnahmevorrichtung mehrere Fotodioden oder mehrere Kameras mit integrierten, (z.B. ebenförmig auf einem "Chip") nebengeordneten optisch-elektrischen Wandlern aufweist, deren optische Achsen einen positiven Winkel miteinander bilden und einen einzelnen Schnittpunkt (= ein Ist-Isozentrum) im Bewegungsvolumen bilden.

Dadurch, dass das Ist-Isozentrum sich mit der Bewegung des Körperteils bewegbar ist, ändert sich das Ausgangsignal (oder die Ausgangsignale) aus der optischen Aufnahmevorrichtung. Die dadurch gebildeten und ermittelten Abweichungen des Ausgangsignals ermöglichen daher mit hoher Genauigkeit eine räumlich dynamische Verfolgung des Körperteils (= des Fluorophors), welche relativ zum Referenzpunkt (z.B. einem Soll-Isozentrum einer Bestrahlungsanlage) erfolgt. Anschließend wird angestrebt, neu entstandene Abweichungen relativ zu einem bisher lokalisierten Ist-Isozentrum dauerhaft zu ermitteln (und sie ggf. durch ein getriggertes Positionierungsmittel zu kompensieren und das Ist-Isozentrum aus dem detektieten Fluorophor als nächstem Soll-Isozentrum dauerhaft bekannt zu halten), so dass der Körperteil mit der optischen Aufnahmevorrichtung immer detektierbar bleibt.

Dabei können u.a. und beispielsweise zwei Messsignale aus einer vom Fluorophor rückstrahlenden Fluoreszenz ermittelt werden, welche über zwei, miteinander schräg geordneten Richtungen aufgenommen werden. Dadurch dass beide Richtungen in einem bekannten räumlichen Koordinatensystem bekannt sind, können aus beiden Messsignalen (welche z.B. über eine Laufzeitmessung oder über eine Aufnahme eines zweidimensionalen, digitalisierten Intensitätsbildes der rückgestreuerten Fluoreszenz) räumliche, zwei- bzw. dreidimensionale Koordinaten der Lage des Fluorophors genau ermittelt werden.

Am geeigneten liegt eine kollimierende Strahlungsrichtung der extrinsischen Lichtquelle mittig zwischen den Richtungen von optischen Achsen der optischen Aufnahmevorrichtung. Weitere Lichtstrahlungen für die extrinsische Lichtquelle können auch verwendet werden, wie z.B. eine ringförmige Beleuchtung, welche über dem zu messenden Bereich liegt. Es können selbstverständlich mehr als eine Lichtquelle oder mehr als zwei optische Achsen für die optische Aufnahmevorrichtung verwendet werden. Damit wird die Genauigkeit oder die Geschwindigkeit erhöht, und es können mögliche lichtabsorbierende Stellen stärker abgelichtet werden, insbesondere um einen im Körper tief gelegten Tumor zu erreichen. Die erfindungsgemäße Vorrichtung kann in Echtzeit funktionieren und 3D-Koordinaten eines Tumors blitzartig geben, z.B. über 50 Aufnahme pro Sekunde. Diese Maß ist abhängig von der verwendeten Technologie der optischen Aufnahmevorrichtung und von der Lichtmenge der rückstrahlenden Fluoreszenz (hängt daher von der Dosierung des Fluorophors sowie dem Wirkungsgrad der extrinsischen Lichtquelle). Sollten die aufgenommen Messsignale zu schwach sein bzw. zu hohe Signal-Rausch-Abstände aufweisen, können mehrere, zeitlich getrennte Messsignale an jeder optischen Achse durchgeführt werden, welche einfach intensitätsmässig statitisch bzw. gemittelt addiert werden. Damit mitteln sich auch vom Vorteil die Rausch-Werten der aufgenommen Bildern, allerdings auf Kosten der Aufnahmegeschwindigkeit. Andere an die optische Aufnahmevorrichtung rückgestrahlte, fluoreszierende Signalwellenlängen als diejenigen des Fluorophors werden ebenfalls optisch gefiltert. Idealerweise weist die Rückstrahlung des Fluorophors zumindest eine Wellenlänge außerhalb des spektralen Bereiches der extrinsichen Lichtquelle auf, damit kein unerwünschtes Licht der Lichtquelle jedoch nur das einzige, vom Fluorophor emittierte und ferner filterbare Licht auf der optischen Aufnahmevorrichtung aufgenommen wird. Es wird ebenfalls aus Interferenzgründen vermieden, dass ein Komponent (Lichtquelle, optische Achse der optischen Aufnahmevorrichtung) der erfindungsgemäßen Vorrichtung im oder entlang von einem/den Strahlgang oder -gängen einer Bestrahlungsanlage (mit z.B. Gamma- oder Protonenstrahlung) stehen. Im Gegensatz werden die Komponenten am weitesten der therapeutischen Strahlung positioniert.

Vorteilhafte Ausgestaltungen der Erfindung sind in den Unteransprüchen dargelegt.

Anschließend wird die Erfindung in einem Ausführungsbeispiel anhand der Zeichnung erläutert.

Dabei zeigt:
- FIG 1: eine erste erfindungsgemäße Vorrichtung in einer radiotherapeutischen Bestrahlungsanlage,
- FIG 2: eine zweite erfindungsgemäße Vorrichtung mit drei Photodioden,
- FIG 3: eine dritte, mit mehreren Photodioden erweiterte erfindungsgemäße,
- FIG 4: eine vierte erfindungsgemäße Vorrichtung mit einer Kamera,
- FIG 5: eine fünfte erfindungsgemäße Vorrichtung mit zwei Kameras.

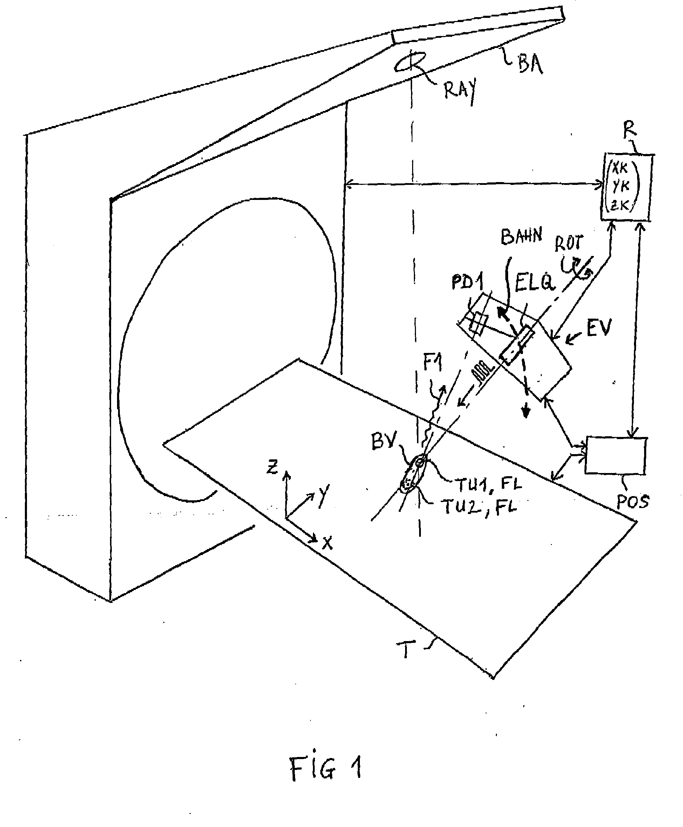

Figur 1 stellt eine Bestrahlungsanlage BA wie ein Linearbeschleuniger dar, deren mindestens ein Strahlausgang RAY bzw. achse einen zu vernichtenden Tumor TU1, TU2 (z.B. im Brust- oder Lungenbereich, jedoch könnten die Extremum-Positionen TU1, TU2 des Tumors an einer anderen Lage des Körpers sein) eines auf einem Tisch T liegenden Menschen (nicht dargestellt) zielt. Durch Atem oder unerwartete Bewegungen des Menschen bewegt sich unvermeidlich der Tumor relativ zum vorgeplanten Strahlungsort (= Isozentrum) im Körper, d.h. es wird bildet ein Bewegungsvolumen gebildet, welches durch die Extremum-Positionen TU1, TU2 des Tumors begrenzt ist. Vor der Bestrahlungstherapie ist es üblich, z.B. eine Computertomographie im Brust- oder Lungenbereich durchzuführen, so dass anschließend eine Neupositionierung der erfindungsgemäßen Vorrichtung EV gegenüber einem großzügig geschätzten Bewegungsvolumen des Tumors erleichtert wird. Dies hilft einem Operator, die erfindungsgemässe Vorrichtung gegenüber dem Patienten zu positionieren, jedoch muss vom Vorteil diese Positionierung nicht hoch genau durchgeführt werden, zumindest reicht es, wenn eine fluoreszierende Strahlung F1 ermittelt werden kann, welche nach einem Einschalten einer extrinsischen Lichtquelle ELQ zur Erregung eines am Tumor angeordneten Fluorophors FL erfolgt und dass ein Messsignal der Erregung an wenigstens einem optisch-elektrischen Wandlern PD1 der erfindungsgemässen Vorrichtung EV ermittelt werden.

Als die extrinsische Lichtquelle ELQ der Vorrichtung EV z.B. in Form einer Welle mit hochfrequenten Pulsen eingechaltet ist, wird beim Eintreffen dieser Welle auf das Fluorophor FL eine fluoreszierende, aus dem an dem Tumor eingebrachten Fluorophors Erregung in Form einer rückstreuenden Welle F1 erzeugt. Diese Welle F1 wird schließlich von der optischen Aufnahmevorrichtung PD1 wellenlängenselektiv ermittelt. In anderen Worten ist eine monodimensionale Abstandmessung zwischen der erfindungsgemäßen Vorrichtung EV und des mit dem Tumor bewegenden Fluorophors FL möglich, nach dem Prinzip eines lichtförmigen "Echos" (= Laufzeitmessung). Die Lichtquelle ELQ und die Photodiode können auch Teile eines Interferometers zur Messung von Entfernungen sein. Es wird dafür z.B. ein Phasenschiebeverfahren oder Weißlichtinterferometrie eingesetzt, wobei ein Kohärenzsgrad zwischen dem Licht aus der Lichtquelle ELQ und dem Licht aus dem Fluorophor gewährleistet sein sollte, um interferenzfähige zu sichern. Damit kann eine monodimensionale Lokalisation des bewegenden Tumors TU1, TU2 in einem Koordinatensystem X, Y, Z erfolgen, welches z.B. ein zum Tisch T festes Koordinatensystem ist. Eine Positionierungseinsteller bzw. -messer POS kennt die Lage des Tisches T gegenüber dem Strahl RAY der Bestrahlungsanlage BA, sowie die Lage der erfindungsgemäßen Vorrichtung EV. D.h. der Lage des Tumors ist nun auch in einer ermittelten Entfernung eines beliebigen Punkts (z.B. des Isozentrums) des Koordinatensystems X, Y, 2 mittels eines Rechners R errechenbar. Dafür ist der Rechner R in Verbindung mit der Bestrahlungsanlage, mit der Positionierungseinheit POS und ggf. mit der erfindungsgemässen Vorrichtung EV z.B. zur Triggerung der Messungen oder zur Verarbeitung der Messergebnissen. Die Positionierungseinheit steuert und ermittelt die Bewegungen bzw. die Lagen der erfindungsgemässen Vorrichtung EV, des Tisches T und ggf. der Bestrahlungsanlage BA. Aus dem Rechner R kann ein Alarmsignal ausgelöst werden, falls eine Unstimmigkeit zwischen einem ermittelten Soll- und dem aktuellen Ist-Isozentrums festgestellt ist. Andererseits können andere Signale ausgelöst werden, um den Strahlgang RAY der Bestrahlungsanlage BA an das ermittelte Soll-Isozentrum rasch einzubringen. Ziel ist selbstverständlich dieses Vorgehen in hochfrequenter Weise durchzuführen, so dass das Ist-Isozentrum den bewegenden Tumor immer übereinstimmt.

Zur weiteren (zwei- oder) dreidimensionalen Lokalisation eines bewegbaren Tumors TU1, ZU2 in einem Bewegungsvolumen BV kann auch die erfindungsgemässe Vorrichtung EV nur die einzelne Photodiode PD1 aufweisen, jedoch in diesem Fall sollte sie auf z.B. eine Schwenkhalterung montiert werden, so dass z.B. für eine dreidimensionale Lokalisation des Tumors über seine Koordinaten XK, YK, ZK in dem Koordinatensystem X, Y, Z wenigstens drei Messungen für drei unterschiedliche Lagen der Photodiode durchführbar wären.

Eine erste Lösung besteht darin, zumindest die Photodiode PD1 bzw. die ganze erfindungsgemässe Vorrichtung EV auf eine Bahn BAHN in einer räumlich bekannten Schrittweise (minimal 3-mal) zu bewegen und für jeden Schritt eine Messung durchzuführen. Diese Bahn BAHN sollte immer zu vom Linearbeschleuniger BA ausgehenden Strahlgängen seitlich angeordnet sein, auch wenn diese Strahlgänge in Bewegung gesetzt werden würden. Auf dieser Bahn BAHN kann die extrinsische Lichtquelle ELQ positioniert werden und ggf. sich mitbewegen.

Eine zweite Lösung besteht darin, die Photodiode PD1 um die optische Achse der extrinsischen Lichtquelle ELQ in einer räumlich bekannten Schrittweise (minimal 3-mal) zu drehen. Dafür kann eine einfache Dreheinrichtung ROT verwendet werden, damit die erfindungsgemässe Vorrichtung EV drehbar ist.

Alternativ könnte die optische Eingangsachse der einzelnen Photodiode PD1 (bzw. die optischen Laufwegen zwischen Tumor und Photodiode) räumlich geändert werden, z.B. durch den Einsatz von Schaltelementen (Spiegeln, Prismen, etc.) zwischen der Photodiode PD1 und dem Tumor TU1, TU2. Diese schaltbaren Elemente sollten idealerweise eine bekannte Schwenkung der optischen Achse der Photodiode PD1 um dem Isozentrum bilden.

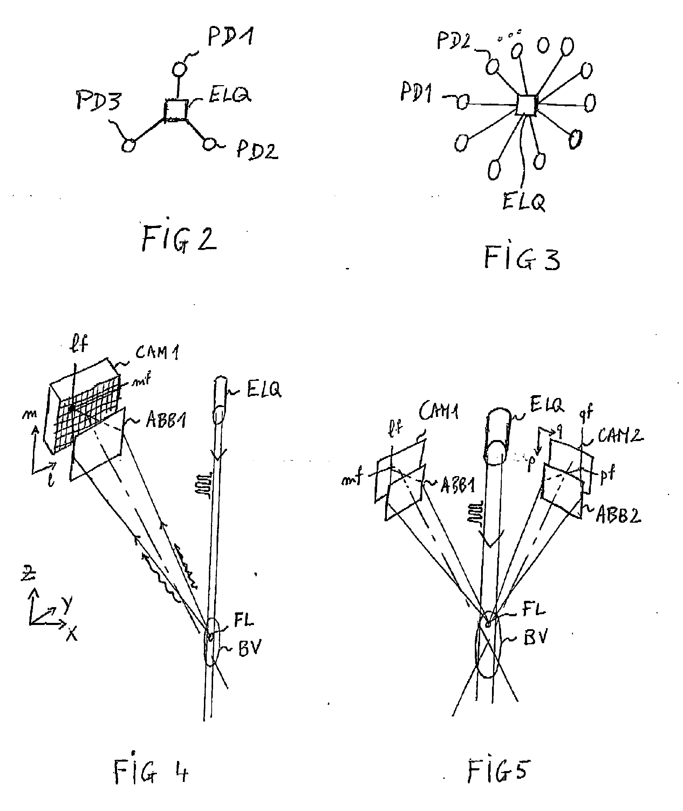

In Figur 2 ist die dargestellte, erfindungsgemässe Vorrichtung EV mit zwei weiteren Photodioden PD2, PD3 erweitert, welche mit der ersten Photodiode PD1 und untereinander nebengeordnet sind-Damit kann ein dreifache Abstandmessung d.h. eine dreidimensionale durchgeführt werden, ohne z.B. eine Photodiode bzw. die erfindungsgemässe Vorrichtung EV zu bewegen oder schwenken. Eine Vorrichtung mit zwei Photodioden PD1, PD2 wäre auch möglich, jedoch benötigt sie zumindest einmal noch eine Bewegung (siehe ROT in Figur 1) oder Schwenkung (siehe BAHN in Figur 1) werden, um eine dreidimensionale Lage des Tumors zu ermitteln.

In Figur 3 ist ein weiteres Ausführungsbeispiel beschrieben, welches eine Erweiterung gemäss Figur 2 bildet. Es werden mehr als drei Photodioden PD1, PD2, etc. im Raum neben der extrinsichen Lichtquelle ELQ angeordnet. Die Lage jeder Photodiode ist im Koordinatensystem X, Y, Z gemäss Figur 1 bekannt, so dass im Koordinatensystem X, Y, Z absolute Entfernungsmessungen zwischen jeder Photodiode und dem Fluorophor immer ermöglicht werden. Einige der Photodioden können an unterschiedlichen Entfernungen von der Lichtquelle ELQ oder/und vom Patienten (d.h. vom Fluorophor) angeordnet werden, so dass z.B. schwache sowie hohe Amplituden der Messsignale an den Photodioden (je nach der Menge des Rücklichtes aus dem Fluorophors) mit einer flexiblen Messdynamik ermittelt werden.

Figur 4 stellt nun ein weiteres Ausführungsbeispiel gemäß Figur 1 dar, wobei anstelle der Photodiode PD1, eine Kamera CAM1 wie eine CCD-Kamera mit in einer Abbildungsebene m, 1 nebengeordneten Pixeln (= optisch-elektrischen Wandlern) in der erfindungsgemässen Vorrichtung EV angeordnet ist. In der Abbildungsebene m, 1 der Kamera CAM1 wird über eine optische Abbildung ABB1 mit einer wegen der Bewegung des Fluorophors ausreichend gewählten Schärfentiefe das Fluorophor abgebildet. Dabei sollte eine Apertur der optischen Abbildung ABB1 so geöffnet werden, dass die Schärfentiefe über dem Bewegungsvolumen BV gesichert wird, jedoch auch nicht zu klein gewählt werden, so dass Lichtverlüste an der Kamera CAM1 vermieden werden. Dieser Aspekt ist wesentlich je nach dem wie tief ein Tumor im Körper sich befindet, da das rückgestrahlte Licht aus einem tiefem Fluorophor (z.B. in der Lunge) mehr absorbiert wird als das Licht aus einem oberflächigen Tumor (z.B. an Augen oder am Gesicht). Nun durch diese geometrische, optische Abbildung, welche im Koordinatensystem X, Y, Z metrisch kalibriert werden kann, können zwei räumliche Koordinaten (z.B. X, Y) mittels der Koordinaten lf, mf des Fluorophors innerhalb der Abbildungsebene m, l ermittelt werden. Die erfindungsgemässe Vorrichtung EV ermöglicht daher eine zweidimensionale Lokalisation des Tumors im Koordinatensystem X, Y, Z. Zur dreidimensionalen Lokalisation des Fluorophors kann gemäss Figur 1 die Kamera CAM1 an festen Positionen bewegt/geschwenkt werden. Es kann ferner ein wellenlängenselektives Filter in der optischen Abbildung ABB1 angeordnet werden, um zum Fluorophor fremdes Licht fern zu halten oder/und es wird eine Kamera verwendet, deren Pixel-Technologie hochempfindliche Aufnahmeeigenschaften im spektralen Bereich des vom Fluorophor zurückkommenden Lichtes hat.

In Figur 5 wird eine Erweiterung der erfindungsgemässen Vorichtung EV gemäss Figur 4 beschrieben, bei welcher eine zu der Kamera CAM1 weitere Kamera CAM2 angeordnet wird, so dass die optische Achsen der Kameras CAM1, CAM2 in Richtung des Köperteils mit dem Fluorophor und dessen Bewegungsvolumen orientiert sind und unter einem positiven Winkel das Bewegungsvolumen eintreffen (die optischen Achsen bilden einen einzelnen, gemeinsamen Schnittpunkt, dessen Abstand mit dem Fluorophor/Tumor ermittelt wird). In anderen Worten triangulieren die Kameras CAM1, CAM2 innerhalb einer Ebene, welche in Nähe des Bewegungsvolumens grob platziert sein sollte. Gemäss dem Ausführungsbeispiel aus Figur 4 ist es nun möglich zu herleiten, dass beide optische Abbildungen ABB1, ABB2 für die beiden, triangulierenden Kamera CAM1, CAM2 liefern zwei geometrisch abgebildete zweidimensionale Lagen mf, lf und pf, qf des Fluorophors/Tumors, aus welchen die dreidimensionalen Koordinaten des Fluorophors/Tumors in dem endgültigen Koordinatensystem X, Y, Z (siehe Figur 1) mittels eines Rechners oder eines vorprogrammierten Bildspeichers errechenbar sind. Damit ist eine einfache und schnelle, dreidimensionale Lokalisation des Tumors gewährleistet. Die beiden optischen Abbildungen ABB1, ABB2 werden derart eingestellt, dass eine punktförmige Abbildung des Fluorophors/Tumors auf jeder Kamera erfolgt, deren laterale Auflösung z.B. zwischen 1/10 und 1/100 des Messbereiches ausreichend ist.
Eine weitere Ausführungsform der erfindungsgemässen Vorrichtung EV wäre auch möglich, indem zu den beiden Kameras CAM1, CM2 weitere Kameras derart angeordnet werden, dass ihre optische Achse sich auf einen einzelnen Schnittpunkt auftreffen. Damit kann man die Lokalisation des Tumors genauer bzw. schneller durchführen.

Allgemeiner können auch die optische Aufnahmevorrichtung PD1, PD2, ... bzw. CAM1, CAM2, ... oder/und die Lichtquelle ELQ auf einer Positionierungseinrichtung wie einer bisherigen kreisbogenförmigen Halterung angeordnet sein, welche die optische Aufnahmevorrichtung oder/und die Lichtquelle seitlich des Wesens positioniert, derart dass Lichtwege zwischen einerseits optischen Eingängen der optischen Aufnahmevorrichtung bzw. optischen Ausgängen der Lichtquelle und andererseits des Körperteils bzw. dessen Bewegungsvolumen minimiert werden. Dadurch wird es gewährleistet, dass eine eingeschränkte Amplitude der Messsignale aus dem Fluorophor ermittelt wird. Daher werden, insbesondere bei konstanter Energiemenge der extrinsichen Lichtquelle ELQ niedrigere Dosiermenge des Fluorophors benötigt oder es werden tiefere (kaum strahlende) Tumoren im Körper visualisierbar sein.

Komplementär -oder Alternativerweise kann die Lichtquelle einen bündelförmigen Strahlausgang aufweisen, dessen Energieverteilung und -dichte entlang eines transversalen Fläche des Strahlausgangs derart eingestellt wird, dass eine Rückstrahlung bzw. -streuung des Fluorophors mit einem ausreichenden Signal-Rausch-Abstand an der optischen Aufnahmevorrichtung gemessen wird. Dies ist besonders geeignet bei schwach rückstrahlenden Tumoren oder wenn ein Tumor wegen seiner Bewegungsbahn im Körper an unterschiedlichen Tiefen im Körper sinkt, jedoch muss für alle unterschiedliche Tiefe noch durch Fluoreszenz sichtbar sein.

Dabei kann auch der bündelförmige Strahlausgang eine longitudinale Hauptachse aufweisen, welche mittels eines hochfrequenten Schwingungselements zur Abtastung des Bewegungsvolumens des Körperteils schwenkbar ist. Damit kann die gebündelte Energie der extrinsischen Lichtquelle ELQ noch konzentrierter an verteilten Orten des Bewegungsvolumen übertragen werden und einen Raster im Bewegungsvolumen (-fläche) des Tumors durch schnelle Abtastungen bilden. Dies vermeidet auch ein Haut- bzw. Weichteilverbrennungseffekt am Patienten, da die Energie nur sehr kurzartig an einer gleichen Stelle der belichteten Haut/Weichteilen bleibt. In diesen Sinnen kann auch die Lichtquelle periodische, pulsförmige Lichtsignale emittieren.

Wie bereits erwähnt weisen die optische Aufnahmevorrichtung bzw. der optische Eingang einer Photodiode/CCD-Kamera Filter zur spektralen Isolierung einer Rückstrahlung bzw. -streuung des Fluorophors auf.

Es kann auch sein, dass die Bestrahlungsanlage die Elektronik der optischen Aufnahmevorrichtung qualitativ stört. Eine Lösung besteht darin, dass der Eingang der optischen Aufnahmevorrichtung über einen Wellenleiter (Glasfaser oder Bündel von Glasfasern) von der Oberfläche des Patienten bis zu den weiter geordneten, optischen Wandlern geführt wird. Damit wird eine Rückstrahlung bzw. -streuung des Fluorophors optimal übertragen, sowie durch Abschirmung der Glasfasern werden zur Fluoreszenz fremde Lichtkomponenten in die erfindungsgemässe Aufnahmevorrichtung nicht eindringen/stören. In anderen Worten entfernt man die elektronisch störbare Komponente der optischen Aufnahmevorrichtung von der Strahlung RAY (siehe Figur 1) bei Hinzufügung eines zwischengeordneten, optischen Wellenleiters.

Es ist auch vom Vorteil zu berücksichtigen, dass die Photodioden bzw. die CCD-Kameras keine hohe Auflösung, jedoch viel mehr eine gute Messdynamik durch möglichst breite, empfindliche Pixel (optisch-elektrische Wandler) benötigen. Durch grosse Pixeln wird eine höhere Menge des Lichtes aufgenommen, was für im Körper tief gelegte Tumoren sehr vorteilhaft ist, weil viel Licht vom Fluorophor im Körper absobiert/gedämpft wird und daher die optische Aufnahmevorrichtung kaum erreichen wird.

Bei der Erfindung ist auch vorausgesetzt, dass die optische Aufnahmevorrichtung mit einer Rechnereinheit mit wenigstens einem Bildspeicher und einer Prozessoreinheit verbunden ist, bei welcher mittels aufgenommener Daten (z.B. Amplitudenwerten an räumlich bekannten Pixeln) der optischen Aufnahmevorrichtung und mittels einer erfassbaren, zu einem bekannten dreidimensionalen Koordinatensystem X, Y, Z relativen Lage der optischen Aufnahmevorrichtung dreidimensionale Koordinaten XK, YK, ZK des Körperteils (Tumors) in dem Koordinatensystem X, Y, Z in Echtzeit ermittelbar sind. Die Rechnereinheit kann ggf. an einem Steuermodul zur berechenbaren Neupositionierung der erfindungsgemäßen Vorrichtung gegenüber dem Körperteil verbunden sein, z.B. um maximale Amplitudenwerte an bestimmten Pixeln der optischen Aufnahmevorrichtung durch sequentielle Bewegung der Vorrichtung zu suchen, als der Patient auf dem Tisch vor der Behandlung liegt. Da die Bewegung oder Position der Vorrichtung relativ zu dem Koordinatensystem X, Y, Z ermittelbar ist, kann mittels einer metrischen Kalibrierung der optischen Komponenten der optischen Aufnahmevorrichtung die dreidimensionale Lage des beleuchtenden Fluorophors (d.h. des Tumors) permanent und genau bekannt gegeben werden, so dass durch Neupositionierung des Patienten der Tumor auch permanent und genau bestrahlt wird.

Bei der Erfindung können sich ungesunde Zellen im Kopf, z.B. an Augen oder am Gesicht, oder im Lungen- oder Brustbereich des Menschen befinden. In anderen Worten ist die Vorrichtung sehr universell für den ganzen Körper einsetzbar. Nur muss je nach der Menge der aus dem Fluorophor rückstreuenden Fluoreszenz die Energie der Lichtquelle variierbar sein.

Die Verwendung der Vorrichtung bildet eine Navigationsmethode für sich oder für eine weitere Planungs-, Sicht- oder Therapeutischen Behandlung. Insbesondere dienen die ermittelbaren dreidimensionalen Koordinaten XK, YK, ZK des Körperteils einer Steuerung einer Bestrahlungsanlage des Körperteils oder einer Unterstützung einer dreidimensionalen Abbildungsanlage des Körpers oder einer Unterstützung eines therapeutischen Planungstools.

Zur Verwendung der Vorrichtung kann außerdem in einfacher Weise der Fluorophor:
- eine Tumoraffinität aufweist und in eine Blutbahn injiziert wird, vorzugsweise in Form von Hematoporphyrin, oder
- in einer eingekapselten Form an einer Stelle des Körperteils angebracht wird, z.B. bei einer Biopsie oder während eines endoskopischen Verfahren, oder
- auf eine flächenförmige Pastille deponiert wird, welche im Bereich der Oberfläche des Körperteils angebracht wird.

Es wurde auch gezeigt, dass eine Verwendung der Vorrichtung gute Ergebnisse zeigt, bei welcher im Erregungsniveau der Fluorophor Lichtwellen im spektralen Bereich 600-760 nm sendet, als die extrinsiche Lichtquelle Licht idealerweise im spektralen Bereich 450-770 nm oder zumindest Pulsiertes Laserlicht bei einer Wellenlänge von 532 nm emittiert und damit den Fluorophor erregt.

Die erfindungsgemässe Vorrichtung hat auch eine sehr attraktive Verwendung, bei welcher sie ein Messkopf für eine Steuerung eines Mechanismus zur Neupositionierung des Wesens in einem absoluten Koordinatensystem ist. In Echtzeit kann die Vorrichtung metrische Daten abgeben, mit welchen z.B. der Tisch mit dem Patient gegenüber dem Strahlgang einer Bestrahlungsanlage neu positioniert wird. Damit wird die Lage des Tumors (d.h. des Fluorophors) mit dem Isozentrum der Bestrahlungsanlage dauerhaft und genau übereinstimmen.

## Patentansprüche

1. Vorrichtung zur räumlichen Lokalisation eines bewegbaren Körperteils (TU1, TU2), welcher sich innerhalb eines Bewegungsvolumens (BV) an der Oberfläche bis auch in Innerem eines lebenden Wesens befindet,
**gekennzeichnet durch**:
- mindestens eine optische Aufnahmevorrichtung, welche außerhalb des Wesens angeordnet ist,
- mindestens ein dosierbarer Fluorophor (FL), welcher im Bereich des Körperteils einbringbar ist,
- eine extrinsische Strahlungsquelle (ELQ), welche außerhalb des Wesens angeordnet ist und aus welcher sich eine Strahlung in Richtung des Bewegungsvolumens ausbreitet, wodurch eine spektralen Erregung des Fluorophors stattfindet, indem eine vom Fluorophor emittierte Welle (F1) erzeugt ist und bei mindestens einer von der optischen Aufnahmevorrichtung messbaren Wellenlänge ermittelbar ist,
- die optische Aufnahmevorrichtung mindestens eine optische Achse aufweist, welche in Richtung des Körperteils und dessen Bewegungsvolumen (BV) orientierbar ist,
- die optische Aufnahmevorrichtung mindestens einen zu der optischen Achse senkrechten optisch-elektrischen Wandler (PD1) aufweist, welcher ein Ausgangsignal abgibt, aus welchem ein Abstand zwischen dem Fluorophor (FL) und einem Referenzpunkt ermittelbar ist.

2. Vorrichtung nach Anspruch 1,
**dadurch gekennzeichnet, dass**
der optisch-elektrische Wandler der optischen Aufnahmevorrichtung eine Fotodiode oder eine Gruppe von nebengeordneten Fotodioden oder Pixeln, wie vorzugsweise in einer Kamera, ist.

3. Vorrichtung nach Anspruch 2,
**dadurch gekennzeichnet, dass** die optische Achse der Fotodiode oder der Kamera bewegbar ist, vorzugsweise mittels einer Schwenkung oder einer Drehung, derart dass die **dadurch** gebildeten, optischen Achsen einen positiven Winkel miteinander bilden und einen einzelnen Schnittpunkt im Bewegungsvolumen bilden.

4. vorrichtung nach Anspruch 1,
**dadurch gekennzeichnet, dass**
die optische Aufnahmevorrichtung mehrere Fotodioden oder mehrere Kameras mit nebengeordneten optisch-elektrischen Wandlern aufweist, deren optische Achse einen positiven Winkel miteinander bilden und einen einzelnen Schnittpunkt im Bewegungsvolumen bilden.

5. Vorrichtung nach einem der Ansprüche 2 oder 4,
**dadurch gekennzeichnet, dass**
die optische Aufnahmevorrichtung oder/und die Lichtquelle auf einer Positionierungseinrichtung angeordnet sind, welche die optische Aufnahmevorrichtung oder/und die Lichtquelle seitlich des Wesens positioniert, derart dass Lichtwege zwischen einerseits optischen Eingängen der optischen Aufnahmevorrichtung bzw. optischen Ausgängen der Lichtquelle und andererseits des Körperteils bzw. dessen Bewegungsvolumen minimiert werden.

6. Vorrichtung nach einem der voranstehenden Ansprüche,
**dadurch gekennzeichnet, dass**
die Lichtquelle einen bündelförmigen Strahlausgang aufweist, dessen Energieverteilung und -dichte entlang eines transversalen Fläche des Strahlausgangs derart eingestellt wird, dass eine Rückstrahlung bzw. -streuung des Fluorophors mit einem ausreichenden Signal-Rausch-Abstand an der optischen Aufnahmevorrichtung gemessen wird.

7. Vorrichtung nach einem der voranstehenden Ansprüche,
**dadurch gekennzeichnet, dass**
mindestens ein optischer Wellenleiter zwischen einem Ausgang der Lichtquelle (ELQ) und dem Bewegungsvolumen (BV) angeordnet ist.

8. Vorrichtung nach einem der voranstehenden Ansprüche,
**dadurch gekennzeichnet, dass**
mindestens ein optischer Wellenleiter zwischen einem Eingang der optischen Aufnahmevorrichtung und dem Bewegungsvolumen (BV) angeordnet ist.

9. Vorrichtung nach einem der voranstehenden Ansprüche,
**dadurch gekennzeichnet, dass**
ein schwingendes Schaltelement in einem Lichtweg zwischen der extrinsichen Lichtquelle und der optischen Aufnahmevorrichtung angeordnet ist.

10. Vorrichtung nach einem der voranstehenden Ansprüche,
**dadurch gekennzeichnet, dass**
die optische Aufnahmevorrichtung Filter zur spektralen Isolierung einer Rückstrahlung bzw. -streuung des Fluorophors aufweist.

11. Vorrichtung nach einem der voranstehenden Ansprüche,
**dadurch gekennzeichnet, dass**
die optische Aufnahmevorrichtung mit einer Rechnereinheit verbunden ist, bei welcher mittels aufgenommener Daten, vorzugsweise zu maximierenden aus dem Fluorophor rückgewonnenen Amplitudenwerten, der optischen Aufnahmevorrichtung und mittels einer erfassbaren, zu einem bekannten dreidimensionalen Koordinatensystem (X, Y, Z) relativen Lage der optischen Aufnahmevorrichtung dreidimensionale Koordinaten (XK, YK, ZK) des Körperteils in dem Koordinatensystem (X, Y, Z) in Echtzeit ermittelbar sind.

12. Verwendung der Vorrichtung gemäß einem der voranstehenden Ansprüche, bei welcher der den Fluorophor umfassende Körperteil durch ungesunde Zellen gebildet ist, beispielsweise wegen eines Tumors oder eines Karzinoms, deren dreidimensionalen Koordinaten (XK, YK, ZK) relativ zu einem bekannten dreidimensionalen Koordinatensystem (X, Y, Z) in Echtzeit ermittelt werden.

13. Verwendung der Vorrichtung nach Anspruch 12, bei welcher sich die ungesunden Zellen im Kopf, z.B. an Augen oder am Gesicht, oder im Lungen- oder Brustbereich des Menschen befinden.

14. Verwendung der Vorrichtung nach Anspruch 12 oder 13, bei welcher die dreidimensionalen Koordinaten (XK, YK, ZK) des Körperteils einer Steuerung einer Bestrahlungsanlage des Körperteils oder einer Unterstützung einer dreidimensionalen Abbildungsanlage des Körpers oder einer Unterstützung eines therapeutischen Planungstools dienen.

15. Verwendung der Vorrichtung nach einem der Ansprüche 12 oder 14, bei welcher der Fluorophor:
- eine Tumoraffinität aufweist und in eine Blutbahn injiziert wird, vorzugsweise in Form vom Hematoporphyrin, oder
- in einer eingekapselten Form an einer Stelle des Körperteils angebracht wird, z.B. bei einer Biopsie oder während eines endoskopischen verfahren, oder
- auf eine flächenförmige Pastille deponiert wird, welche im Bereich der Oberfläche des Körperteils angebracht wird.

16. Verwendung der Vorrichtung nach einem der Ansprüche 12 oder 15, bei welcher der Fluorophor Lichtwellen im spektralen Bereich 600-760 nm in Erregungsniveau sendet, als die extrinsiche Lichtquelle Licht idealerweise im spektralen Bereich 450-770 nm oder zumindest Pulsiertes Laserlicht bei einer Wellenlänge von 532 nm emittiert und damit den Fluorophor erregt.

17. Verwendung der Vorrichtung gemäss einem der voranstehenden Ansprüche, wobei die Vorrichtung ein Messkopf für eine Steuerung eines Mechanismus zur Neupositionierung des Wesens in einem absoluten Koordinatensystem ist.
